# EUROPÄISCHE PATENTSCHRIFT

(11) **EP 2 134 258 B1**
(45) Veröffentlichungstag und Bekanntmachung des Hinweises auf die Patenterteilung: **11.12.2013**
(21) Anmeldenummer: 08734654.0
(22) Anmeldetag: 19.03.2008
(51) Int. Cl.: A61B 5/151, A61B 5/145

(54) **STECHSYSTEM**
PIERCING SYSTEM
SYSTÈME DE PIQÛRE

(30) Priorität: 12.04.2007 EP 07007470
(43) Veröffentlichungstag der Anmeldung: 23.12.2009
(73) Patentinhaber: Roche Diagnostics GmbH, 68305 Mannheim (DE); F. Hoffmann-La Roche AG, 4070 Basel (CH)
(72) Erfinder: HARTTIG, Herbert, 67434 Neustadt (DE); KONYA, Ahmet, 67165 Waldsee (DE); SCHÖTTLE, Klaus, 77731 Willstätt (DE)
(74) Vertreter: Twelmeier Mommer & Partner
(86) Internationale Anmeldenummer: PCT/EP2008/002172
(87) Internationale Veröffentlichungsnummer: WO 2008/125178

(56) Entgegenhaltungen:
- EP-A1- 1 360 935
- EP-A1- 1 967 139
- EP-A1- 1 974 667
- WO-A-2005/104948
- WO-A-2005/107596
- WO-A1-2008/083844
- DE-B1- 2 803 345

## Beschreibung

Die Erfindung geht aus von einem Stechsystem zum Gewinnen einer Körperflüssigkeitsprobe. Zu einem derartigen Stechsystem gehören ein Magazin, das einen mehrere Lanzetten tragenden Lanzettenträger enthält, und ein Stechgerät mit einem Fach für ein derartiges Magazin, einem Fortschaltmechanismus, um die Lanzetten eines in das Fach eingesetzten Magazins nacheinander in eine Stechposition zu bringen, und einem Stechantrieb, um eine in der Stechposition positionierte Lanzette für eine Stichbewegung zu beschleunigen.

Ein derartiges Magazin, das die Oberbegriff des Anspruchs 1 angegebenen Merkmale aufweist, ist aus der WO 2005/107596 A bekannt. Bei einem Stich wird eine auf dem Trägerband in dessen Längsrichtung orientierte Lanzette zusammen mit einer Führungsrolle des Trägerbands in Stichrichtung bewegt.

Aus der EP 1 360 935 A1 ist ein ähnliches Stechsystem mit einem Trägerband bekannt, auf dem Lanzetten in dessen Längsrichtung orientiert sind. Bei einem Stich führt das gesamte Magazin die Stichbewegung aus und wird relativ zum Stechgerät bewegt.

Aus der WO 2005/104948 A ist ein Stechsystem mit einem Magazin bekannt, das ein Trägerband mit Lanzetten enthält, die quer zur Längsrichtung des Trägerbands orientiert sind. Für einen Stich wird eine Lanzette mit einer Aktuatorik des Stechgeräts von dem Trägerband entfernt.

Aus der DE 28 03 345 B1 ist ein Stechsystem ohne Magazine bekannt, bei dem Lanzetten auf einem Trägerband quer zu dessen Längsrichtung orientiert sind.

Derartige Stechsysteme werden beispielsweise von Diabetikern verwendet, die mehrmals täglich ihren Blutzuckerspiegel überprüfen müssen und dafür eine Körperflüssigkeitsprobe, in der Regel Blut oder interstitielle Flüssigkeit, benötigen, die durch eine mit einem Stechsystem erzeugte Stichwunde gewonnen wird.

Bei derartigen Stechsystemen muss durch eine geeignete Kopplung eine Kraftübertragung von dem Stechantrieb auf eine in der Stechposition positionierte Lanzette erfolgen. Bekannte Kopplungen sind komplex, mechanisch aufwendig und machen eine Fertigung mit geringen Toleranzen erforderlich, so dass erhebliche Kosten entstehen.

Aufgabe der Erfindung ist es deshalb, einen Weg aufzuzeigen, wie ein kostengünstiges Stechsystem der eingangs genannten Art geschaffen werden kann.

Diese Aufgabe wird durch ein Magazin mit den im Anspruch 1 angegebenen Merkmalen gelöst, das Teil eines, Stechsystems nach Anspruch 3 sein kann. Vorteilhafte Weiterbildungen der Erfindung sind Gegenstand der Unteransprüche.

Bei einem erfindungsgemäßen Stechsystem koppelt der Stechantrieb nicht unmittelbar an eine Lanzette an. Stattdessen erfolgt die Kopplung des Stechantriebs an eine in der Stechposition positionierte Lanzette über eine Kopplungseinrichtung des Magazins, die im Betrieb den Stechantrieb mit einer in der Stechposition positionierten Lanzette koppelt. Auf diese Weise lässt sich der mechanische Aufwand zur Kopplung des Lanzettenantriebs mit der für einen Einstich bestimmten Lanzette reduzieren.

Beispielsweise kann die Kopplungseinrichtung des Magazins bereits durch das Einlegen des Magazins in das Magazinfach des Stechgeräts an den Stechantrieb gekoppelt werden. Die korrekte Positionierung der Kopplungseinrichtung in Bezug auf eine in der Stechposition positionierte Lanzette oder den Lanzettenträger kann bereits bei der Fertigung des Magazins vorgenommen werden, so dass die Ankopplung der Kopplungseinrichtung des Magazins an die Lanzette ohne zutun eines Benutzers möglich ist. Insbesondere bei bandförmigen Lanzettenträgem lässt sich auf diese Weise mit besonders geringerem Aufwand eine zuverlässige Ankopplung an den Stechantrieb ermöglichen, so dass diese eine Lanzette für eine Einstichbewegung beschleunigen kann.

Bevorzugt koppelt die Kopplungseinrichtung mit dem Lanzettenträger, so dass bei einer Stichbewegung der Lanzettenträger zusammen mit der für einen Stich bestimmten Lanzette bewegt wird. Über den Lanzettenträger lässt sich mechanisch besonders einfach eine Übertragung der von einem Lanzettenantrieb erzeugten Antriebskraft von der Kopplungseinrichtung auf die in der Stechposition positionierte Lanzette bewirken. Der Lanzettenträger kann nämlich relativ zu der Kopplungseinrichtung auch bei einem Betätigen des Fortschaltmechanismus, mit dem frische Lanzetten des Lanzettenträgers in die Stechposition befördert werden, eine definierte Position beibehalten. Die Kopplungseinrichtung kann deshalb mit relativ geringem Aufwand nach jeder Betätigung des Fortschaltmechanismus für einen weiteren Stich an den Lanzettenträger ankoppeln oder sogar dauerhaft mit dem Lanzettenträger gekoppelt sein, also auch während einer Betätigung des Fortschaltmechanismus mit dem Lanzettenträger gekoppelt bleiben. In ähnlicher Weise kann auch zwischen der Kopplungseinrichtung des Magazins und dem Stechantrieb mit relativ geringem Aufwand eine zuverlässige Kopplung realisiert werden, da durch den Fortschaltmechanismus bewirkte Bewegungen des Lanzettenträgers ohne Einfluss auf den Stechantrieb und die Kopplungseinrichtung des Magazins bewirkt werden können. Vorteilhaft ist es auf diese Weise nicht erforderlich, nach jedem Einstich eine aufwändige Repositionierung der an der Ankopplung des Stechantrieb beteiligten Teile vorzunehmen oder durch komplexes und kostenintensive mechanische Maßnahmen für eine entsprechend exakte Positionierung und Ankopplung nach jedem Betätigen des Fortschaltmechanismus zu sorgen.

Weitere Einzelheiten und Vorteile der Erfindung werden anhand von Ausführungsbeispielen unter Bezugnahme auf die beigefügten Zeichnungen erläutert. Die dabei beschriebenen Merkmale können einzeln und in Kombination zum Gegenstand von Ansprüchen gemacht werden. Gleiche und einander entsprechende Bauteile verschiedener Ausführungsbeispiele sind in den Zeichnungen mit übereinstimmenden Bezugszeichen gekennzeichnet. Es zeigen:
- Figur 1: eine schematische Darstellung eines Ausführungsbeispiels eines Magazins, das einen bandförmigen Lanzettenträger mit mehreren Lanzetten enthält, in einer Draufsicht;
- Figur 2: ein Ausführungsbeispiel eines Lanzettenträgers mit Lanzetten und Testfeldern;
- Figur 3: eine Seitenansicht zu Figur 1 vor einem Einstich;
- Figur 4: eine Darstellung gemäß Figur 2 während eines Einstichs;
- Figur 5: eine schematische Detailansicht zur Figur 3;
- Figur 6: einen Ausschnitt eines weiteren Ausführungsbeispiels eines Magazins in einer Schnittdarstellung;
- Figur 7: eine schematische Detailansicht eines weiteren Ausführungsbeispiels einer Kopplungseinrichtung eines Magazins mit einem bandförmigen Lanzettenträger;
- Figur 8: eine Detailansicht gemäß Figur 7 bei einem Einstich;
- Figur 9: eine Schrägansicht zu Figur 7; und
- Figur 10: ein Ausführungsbeispiel eines Stechgeräts.

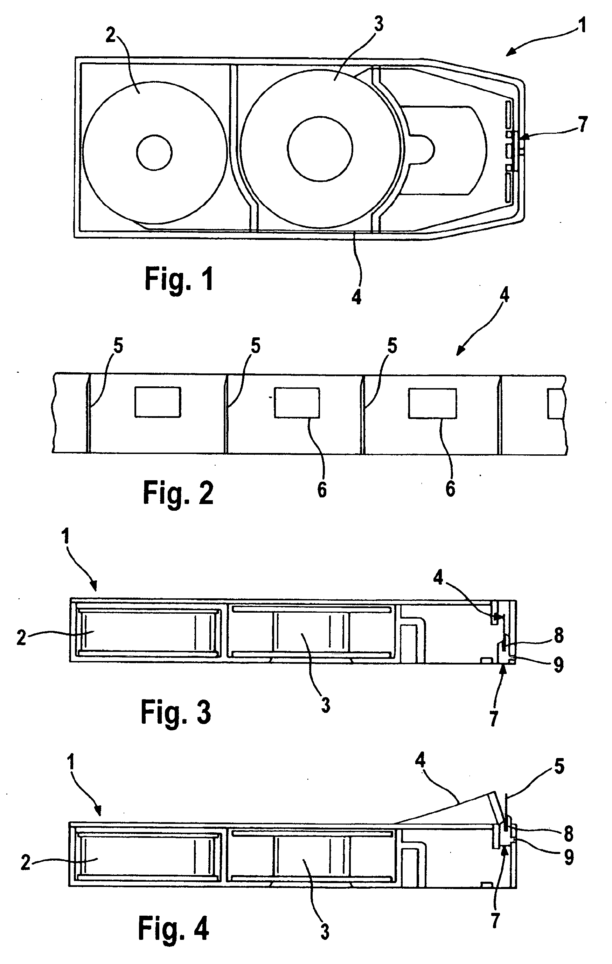

Figur 1 zeigt ein Magazin 1, das zwei Rollen 2, 3 enthält, auf die ein bandförmiger Lanzettenträger 4 aufgewickelt ist, der schematisch in Figur 2 dargestellt ist. Die erste Rolle 2 trägt dabei Abschnitte des Lanzettenträgers 4 mit unbenutzten Lanzetten 5 und die zweite Rolle 3 verbrauchte Abschnitte des Lanzettenträgers 4. Ist das dargestellte Magazin 1 in ein Magazinfach eines Stechgeräts (nicht dargestellt) eingesetzt, wird die zweite Rolle 3, auf die verbrauchte Abschnitte des Lanzettenträgers 4 aufgewickelt werden, von einem Fortschaltmechanismus des Stechgeräts angetrieben, um die in dem Magazin 1 enthaltenen Lanzetten 5 nacheinander in eine Stechposition zu befördern. In der Stechposition ist eine Lanzette 5 so positioniert, dass sie bei einer von dem Stechantrieb des Stechgeräts bewirkten Stichbewegung in einem angepressten Körperteil eines Benutzers eines Stichwunde erzeugt, aus der eine Körperflüssigkeitsprobe für diagnostische Zwecke gewonnen werden kann. Durch Betätigen des Fortschaltmechanismus (nicht dargestellt) kann die angetriebene Rolle 3 des Magazins 1 soweit gedreht werden, dass für einen Stich eine frische Lanzette 5 des Lanzettenträgers 4 in die Stechposition gelangt.

Gemäß Figur 2 sind die Lanzetten 5 auf dem bandförmigen Lanzettenträger quer zu dessen Längsrichtung angeordnet. Zwischen zwei Lanzetten 5 befindet sich jeweils ein Testfeld 6 zur Untersuchung einer aus einer Stichwunde gewonnenen Körperflüssigkeitsprobe. Die Testfelder 6 können zur photometrischen Bestimmung einer Analytkonzentration Testchemikalien enthalten, die eine konzentrationsabhängige Verfärbung bewirken. Möglich ist es auch, die Testfelder 6 für eine elektrochemische oder spektroskopische Untersuchung einer Körperflüssigkeitsprobe einzurichten.

Das in Figur 1 in einer Draufsicht und in den Figuren 3 und 4 in einer Seitenansicht dargestellte Magazin 1 weist eine Kopplungseinrichtung 7 auf, relativ zu der die von dem Lanzettenträger 4 getragenen Lanzetten 5 durch Betätigen des Fortschaltmechanismus beweglich sind und die im Betrieb den Stechantrieb eines Stechgeräts mit einer in der Stechposition positionierten Lanzette 5 koppelt, um bei einem Stich eine von dem Stechantrieb 11 erzeugte Antriebskraft auf die in der Stechposition positionierte Lanzette 5 zu übertragen. Bei dem dargestellten Ausführungsbeispiel koppelt die Kopplungseinrichtung 7 den Stechantrieb eines Stechgeräts mit dem Lanzettenträger 4, so dass dieser bei einem Stich zusammen mit einer in der Stechposition positionierten Lanzette 5 bewegt wird.

Die Kopplungseinrichtung 7 weist eine Aufnahme 8, bei dem dargestellten Beispiel in Form einer Nut, für den Lanzettenträger 4 auf. Die Aufnahme 8 ist Teil eines in Einstichrichtung relativ zu einem Magazingehäuse verfahrbaren Schlittens. Über die Aufnahme 8 ist die Kopplungseinrichtung 7 ständig mit dem Lanzettenträger 4 gekoppelt. Vorteilhaft bleibt die Kopplungseinrichtung 7 deshalb auch während einer Betätigung des Fortschaltmechanismus mit dem Stechantrieb gekoppelt, so dass eine mechanische Abstimmung mit dem Fortschaltmechanismus nicht erforderlich ist.

Bei einem Stich wird die Kopplungseinrichtung 7 in einer Vorschubphase von dem Lanzettenantrieb in Einstichrichtung aus der in Figur 3 dargestellten Position in die in Figur 4 dargestellte Position verfahren und bei einer anschließenden Rückführbewegung zurück in die in Figur 3 dargestellte Position bewegt. Zum Ankoppeln an den Stechantrieb weist die Kopplungseinrichtung 7 ein Kopplungselement 9 auf, bei dem dargestellten Ausführungsbeispiel in Form eines Zapfens, das formschlüssig mit einem dazu passenden Kopplungselement des Stechantriebs in Eingriff tritt.

Bei einem Stich bewirkt die von einem angekoppelten Stechantrieb erzeugte Antriebskraft eine Bewegung der Kopplungseinrichtung 7 einschließlich des in der Nut 8 positionierten Bandabschnitts des Lanzettenträgers 4 in Einstichrichtung. Eine nicht dargestellte Biegeeinrichtung, die wahlweise an dem Magazin oder an dem Stechgerät angeordnet sein kann, kann dabei ein Biegen des Lanzettenträgerbandes 4 gemäß Figur 4 bewirken, so dass sich die Spitze der in der Stechposition positionierten Lanzette 5 von der Oberfläche des bandförmigen Lanzettenträgers 4 abhebt und, ohne durch den bandförmigen Lanzettenträger 4 behindert zu werden, in ein angelegtes Körperteil eines Benutzers einstechen kann. Die Biegeeinrichtung kann beispielsweise als zwei schräg zur Stichrichtung stehende Gabelzinken ausgebildet sein, zwischen denen eine Lanzette bei einem Einstich hindurchragt, wobei die Gabelzinken beidseitig neben der Lanzette das Lanzettenträgerband zurückhalten und abbiegen.

Nach einem Stich kann durch Betätigen des Fortschaltmechanismus eines Stechgeräts ein Testelement 6 des bandförmigen Lanzettenträgers 4 in der Nut 8 der Kopplungseinrichtung 7 positioniert werden. Durch erneutes Betätigen des Stechantriebs kann dann das Testfeld 6 zu einer durch einen vorgehenden Stich erzeugten Stichwunde verschoben und von dem Testfeld 6 eine Körperflüssigkeitsprobe aufgenommen werden.

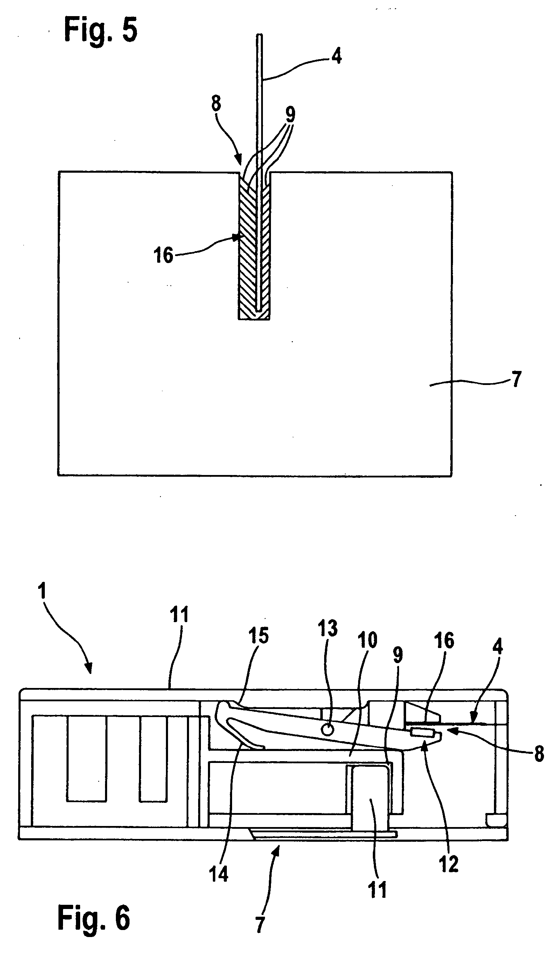

Figur 5 zeigt schematisch ein Detail der Kopplungseinrichtung 7 des vorhergehend beschriebenen Ausführungsbeispiels. Wie man darin sieht, sind die Wände der als Nut ausgebildeten Aufnahme 8 mit schräg stehenden Fasern, beispielsweise Borsten 9, besetzt. Die Borsten 9 sind dabei derart angeordnet, dass sie mit ihrem freien Ende schräg in Rückführrichtung ausgerichtet sind. Auf diese Weise wird der Lanzettenträger 4 bei einer Rückführbewegung der Kopplungseinrichtung 7 von den Borsten 9 gehalten und kann besser zusammen mit der Kopplungseinrichtung 7 von einem angekoppelten Stechantrieb zurückgezogen werden. Die Kopplungseinrichtung 7 tritt bei dem dargestellten Ausführungsbeispiel mit dem Lanzettenträger 4 also über eine strukturierte Kopplungsfläche in Kontakt, die einer Relativbewegung zwischen Lanzettenträger 4 und Kopplungsfläche einen von der Richtung der Relativbewegung abhängigen Widerstand entgegen setzt.

Eine derartige Kopplungsfläche behindert den durch den Fortschaltmechanismus bewirkten Transport des Lanzettenträgers 4 nicht oder nur geringfügig, da die strukturierte Oberfläche nur in Richtung der Rückführbewegung eine erhöhte Reibung bewirkt. Bei der Rückführbewegung bewirkt die strukturierte Oberflache der Kopplungsflächen der Aufnahme 8, bei dem dargestellten Ausführungsbeispiel also der gegenüberliegenden Seitenwände der Nut 8, jedoch verbesserte Haftung des Lanzettenträgers 4 in der Aufnahme 8, so dass der Lanzettenträger 4 schnell zusammen mit der Kopplungseinrichtung 7 zurückgezogen und eine von dem Lanzettenträger 4 getragene Lanzette 5 aus einer soeben erzeugten Stichwunde gezogen werden kann.

Eine strukturierte Oberfläche mit derartig vorteilhaften Eigenschaften lässt sich nicht nur durch die in Figur 4 dargestellten Fasern 9, sondern auch durch andere Vorsprünge, Erhebungen oder Vertiefungen der Kopplungsfläche der Aufnahme 8, die mit der Oberfläche des Lanzettenträgers 4 in Kontakt treten, erreichen.

Eine strukturierte Oberfläche gemäß Figur 5 kann beispielsweise durch eine Beschichtung mit parallel orientierten Stapelfasern erreicht werden. Die Fasern 9 werden in einer Fixierschicht befestigt. Die stehen dabei gegenüber der Kopplungsfläche der Aufnahme 8 in einem Winkel zwischen 30° und 90°, bevorzugt 45° bis 75°. Die Fasern sind dabei zum Boden der Nut hin, also in Richtung der Rückführbewegung und damit jene Richtung geneigt, in der auf den Lanzettenträger 4 von der Kopplungsfläche 8 eine Kraft ausgeübt werden soll, wie es in Figur 5 dargestellt ist.

Der Transport des Lanzettenträgers 4 zwischen zwei derartig strukturierten Kopplungsflächen erfolgt mit vorteilhaft geringer Reibung. Auch das Einlegen eines bandförmigen Lanzettenträgers 4 in die Aufnahme 8 bedingt nur minimale Reibung. Wird aber versucht, den Lanzettenträger 4 gegen die Neigungsrichtung der Fasern 9 zu bewegen, so verspreizen sich diese selbst an kleinsten Unebenheiten und halten so den Lanzettenträger 4 zurück. Als strukturierte Kopplungsfläche mit geneigten Fasem 9 kann beispielsweise eine Flockfolie verwendet werden, die auf die Kopplungseinrichtung aufgebracht, insbesondere aufgeklebt wird.

Eine geeignete Flockfolie kann beispielsweise folgendermaßen hergestellt werden. Eine Polyesterfolie, beispielsweise aus Hostaphan RN50 von Mitsubishi Polyester Film, Wiesbaden, wird mit einem leitfähigen Kleber, beispielsweise Mecoflock D453/5-09 von Kissel + Wolf GmbH, Wiesloch, beschichtet. Die Klebstoffschicht wird beispielsweise mit einem Ero-Mini-Flockgerät der Firma Maag Flockmaschinen GmbH, Gommeringen, mit Polyamid-Flock von 1,7 dtex und 0,5 mm Länge geflockt. Derartiger Flock ist von der Firma Swissflock, Stuttgart erhältlich. Unmittelbar nach der Beflockung wird die Folie durch einen Kalander gezogen, bei dem der Walzenspalt beispielsweise auf 360 µm eingestellt ist. Dadurch legen sich die Fasern schräg. Nach dem Trocknen wird die Flockschicht abgesaugt und so von losen Fasern gereinigt. In Walzrichtung haben die Fasern dann einen Winkel zur Polyesterfolie von etwa 45°. Senkrecht zur Walzrichtung sind Fasern in Mittel senkrecht ausgerichtet.

Eine weitere Möglichkeit zur Herstellung einer geeigneten Flockfolie ist es, eine Kunststofffolie in Abständen von beispielsweise 10 mm wechselseitig so nach oben und unten parallel zu knicken, dass die Knicke jeweils Winkel von 100° bis 140°, beispielsweise 120°, einschließen. Wie in dem vorhergehenden Beispiel wird die Folie dann beflockt, aber nicht durch einen Walzenspalt transportiert. Die Fasern werden nämlich bedingt durch die Neigung der Folienabschnitte schräg in den Kleber eingeschossen und haben so gegenüber der Folie jeweils einen Winkel von etwa 60 bis 70°.

Der Schlitz, der in Figur 5 dargestellten Kopplungseinrichtung 7 kann beispielsweise eine Breite von 0,8 mm haben. Die Wände des Schlitzes können beispielsweise mit einem Epoxidkleber mit Flockfolie beklebt werden, so dass die Fasern 9 gemäß Figur 5 in Richtung auf den Boden des Schlitzes zeigen.

In Figur 6 ist ein weiteres Ausführungsbeispiel eines Magazins 1 mit einer Kopplungseinrichtung 7 dargestellt. Das in Figur 6 dargestellte Magazin 1 enthält ebenso wie das in Figur 1 dargestellte Magazin 1 einen bandförmigen Lanzettenträger 4 mit mehreren Lanzetten 5 in einer Anordnung gemäß Figur 2 und unterscheidet sich von dem im vorhergehenden beschriebenen Ausführungsbeispiel im wesentlichen nur durch die Ausbildung der Kopplungseinrichtung 7, die im Betrieb den Stechantrieb eines Stechgeräts mit dem Lanzettenträger 4 koppelt, um bei einem Stich eine von dem Stechantrieb erzeugte Antriebskraft auf eine in der Stechposition positionierte Lanzette 5 zu übertragen.

Die Kopplungseinrichtung 7 umfasst einen Schlitten 10, der relativ zu einem Magazingehäuse 11 verschiebbar gelagert ist. Der Schlitten 10 weist als Kopplungselement zur Ankopplung an den Stechantrieb eines Stechgeräts eine Ausnehmung 9 auf, in die der Stechantrieb 11 mit einem Zapfen eingreift, so dass der Schlitten 10 durch eine von dem Stechantrieb 11 erzeugte Antriebskraft in Einstichrichtung verschoben werden kann.

Zur Kopplung an den Lanzettenträger 4 weist die dargestellte Kopplungseinrichtung 7 eine Aufnahme 8 auf, durch die der Lanzettenträger 4 hindurchgeführt ist. Die Aufnahme 8 wird von einer an dem Schlitten 10 starr angeordneten Kopplungsfläche 16 und einem relativ zu der Kopplungsfläche 16 beweglichen Andruckelement 12 gebildet, das den Lanzettenträger 4 bei einem Stich in der Aufnahme klemmt und nach einer erfolgten Einstich- und Rückführbewegung wieder freigibt.

Das Andruckelement 12 ist auf dem Schlitten 10 um einen Drehpunkt 13 drehbar gelagert. Ein Federelement 14 presst das hintere Ende des Andruckelements 12 gegen eine Kulisse 15, die bei dem dargestellten Ausführungsbeispiel in das Magazingehäuse 11 integriert ist. Eine Bewegung des Schlittens 10 in Einstichrichtung bewirkt, dass das Andruckelement 12 mit seinem hinteren Ende die Kulisse 15 abfährt und wegen seiner drehbaren Lagerung dadurch eine Schließbewegung ausführt, die sein vorderes Ende gegen eine Kopplungsfläche 16 des Schlittens 10 drückt, so dass der Lanzettenträger 4 zwischen der Kopplungsfläche 16 und dem vorderen Ende des Andruckelements 12 eingeklemmt wird. Auf diese Weise fasst die Kopplungseinrichtung 7 des dargestellten Ausführungsbeispiels den Lanzettenträger 4 bei einem Stich klemmend und gibt ihn nach erfolgter Einstich- und Rückführbewegung wieder frei. Durch das Einklemmen des Lanzettenträgers 4 in der Aufnahme 8 kann dieser bei einer Rückführbewegung rasch und zuverlässig wieder zurückgezogen werden, so dass eine von ihm getragene Lanzette aus einer erzeugten Stichwunde schnell wieder herausgezogen werden kann, was für einen möglichst schmerzarmen Einstich wichtig ist.

Die Klemmkraft zum Einklemmen des Lanzettenträgers 4 wird bei dem dargestellten Ausführungsbeispiel durch das Federelement 14 bewirkt, welches das hintere Ende des Andruckelements 12 gegen die Kulisse 15 drückt, welche bei einer Einstichbewegung die Schließbewegung des Andruckelements 12 vorgibt.

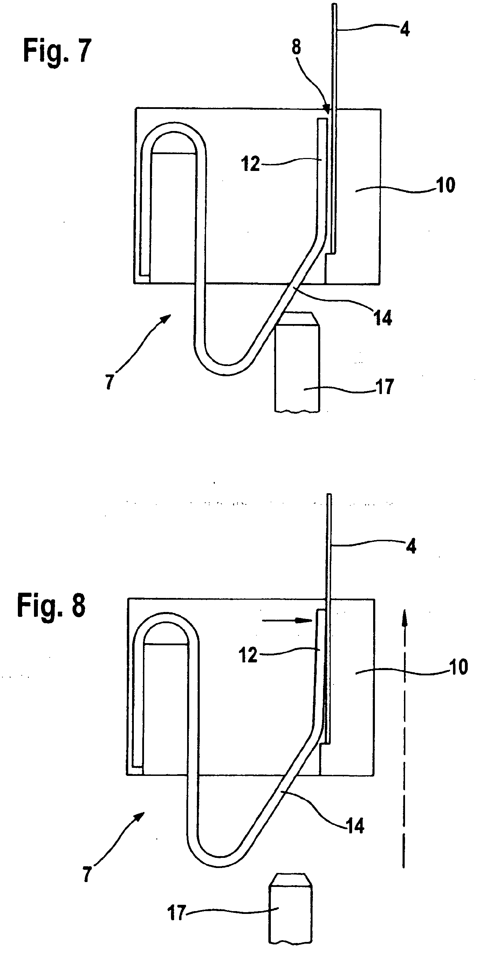

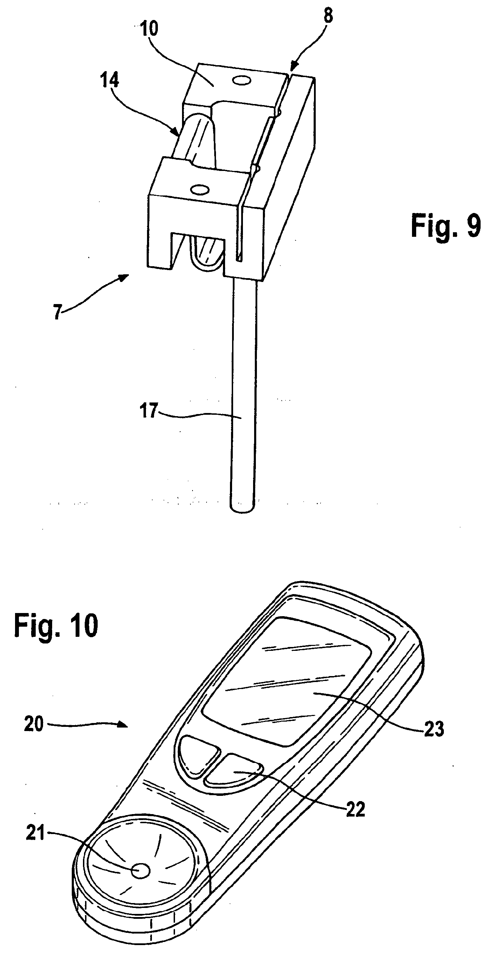

In den Figuren 7 bis 9 ist ein weiteres Ausführungsbeispiel einer Kopplungseinrichtung 7 dargestellt, die beispielsweise bei einem Magazin 1 gemäß den Figuren 1 oder 6 anstelle der dargestellten und vorhergehend beschriebenen Kopplungseinrichtungen 7 verwendet werden kann. Ähnlich wie bei der in Figur 6 dargestellten Kopplungseinrichtung 7 fasst die in den Figuren 7 bis 9 dargestellte Kopplungseinrichtung 7 den Lanzettenträger 4 bei einem Stich klemmend und gibt ihm nach einer erfolgten Einstich- und Rückführbewegung wieder frei. In Figur 7 ist die Kopplungseinrichtung 7 vor einem Einstich also mit einem freigegebenen Lanzettenträger 4 dargestellt. In Figur 8 ist der Lanzettenträger 4 von der Kopplungseinrichtung 7 eingeklemmt, also gemäß den bei einem Einstich auftretenden Verhältnissen dargestellt. Figur 9 zeigt eine Schrägansicht zu dem in den Figuren 7 und 8 dargestellten Ausschnitt der Kopplungseinrichtung 7.

Die Kopplungseinrichtung 7 des in den Figuren 7 bis 9 dargestellten Ausführungsbeispiels umfasst ähnlich wie das in Figur 6 dargestellte Ausführungsbeispiel einen Schlitten 10, der in Einstichrichtung verfahrbar ist und eine Aufnahme 8 trägt, durch die der Lanzettenträger 4 hindurchgeführt ist. Die Einstichrichtung ist in Figur 8 durch einen gestrichelten Pfeil angedeutet. Die Aufnahme 8 für den Lanzettenträger 4 kann beispielsweise als Nut ausgebildet sein.

Die Aufnahme 8 wirkt mit einem Andruckelement 12 zusammen, das bei einem Stich den Lanzettenträger 4 gegen eine Kopplungsfläche der Aufnahme 8 drückt und auf diese Weise einklemmt. Die Klemmkraft zum Einklemmen des Lanzettenträgers 4 wird von einem Federelement 14 bewirkt, das bei dem dargestellten Ausführungsbeispiel einstückig mit dem Andruckelement 12 ausgebildet ist. Das Federelement 14 wirkt mit einem Öffner 17 zusammen, der bei dem dargestellten Ausführungsbeispiel ortsfest in Bezug auf das Magazingehäuse (nicht dargestellt) angeordnet ist und beispielsweise als ein Vorsprung an dem Magazingehäuse ausgebildet werden kann.

Befindet sich der Schlitten 10 vor einem Einstich in seiner in Figur 7 dargestellten Ausgangsposition, drückt der Öffner 17 gegen das Federelement 14 und bewirkt auf diese Weise eine Öffnung der Aufnahme 8. Der Lanzettenträger 4 kann dann durch Betätigen des Fortschaltmechanismus (nicht dargestellt) senkrecht zur Einstichrichtung in der Aufnahme 8 bewegt werden. Wird der Schlitten 10 in Einstichrichtung bewegt, entfernt sich das Federelement 14 von dem Öffner 17, so dass es den Lanzettenträger 4 in der Aufnahme 8 einklemmt. Durch die von dem Federelement 14 ausgeübte Federkraft wird der Lanzettenträger 4 auf diese Weise gegen die Kopplungsfläche der Aufnahme 8 gedrückt.

Das Federelement 14 kann kostengünstig aus Kunststoff hergestellt werden. Möglich ist es aber auch, das Federelement 14 aus Metall herzustellen.

Figur 10 zeigt ein Ausführungsbeispiel eines Stechgeräts zum Gewinn einer Körperflüssigkeitsprobe. Das Stechgerät 20 weist eine Geräteöffnung 21 auf, gegen die ein Körperteil zum Erzeugen einer Stichwunde gepresst wird. Das Stechgerät 20 umfasst ferner Bedienungselemente 22 in Form von Tasten und eine Anzeigeeinrichtung 23 in Form einer Flüssigkristallanzeige zum Anzeigen von Untersuchungsergebnissen. Das Stechgerät 20 hat ein Aufnahmefach (nicht dargestellt) für ein Magazin 1, das einen mehrere Lanzetten 5 tragenden Lanzettenträger 4 enthält. Ausführungsbeispiele eines derartigen Magazins 1 wurde vorhergehend anhand der Figuren 1 bis 9 erläutert. Das Aufnahmefach des dargestellten Stechgeräts 20 hat eine verschließbare Öffnung, die sich auf der Rückseite des dargestellten Stechgeräts 20.

In das dargestellte Stechgerät 20 ist eine Mess- und Auswerteeinrichtung (nicht dargestellt) integriert, mit der die Analytkonzentration einer Körperflüssigkeitsprobe bestimmt werden kann. Hierzu wird eine Körperflüssigkeitsprobe von einem Testfeld 6 des in einem eingesetzten Magazin 1 enthaltenen Lanzettenträgerbandes 4 (siehe Figur 2) aufgenommen, indem nach Erzeugen einer Stichwunde ein Testfeld 6 des Lanzettenträgerbandes 4 durch Betätigen des Fortschaltmechanismus unter der Geräteöffnung 21 positioniert wird. Durch erneutes Betätigen des Stechantriebs kann der Lanzettenträger 4 mit dem Testfeld 6 dann in Einstichrichtung verfahren werden, so dass das Testfeld 6 zum Aufnehmen einer Körperflüssigkeitsprobe an der Stichwunde eines an die Geräteöffnung 21 angepressten Körperteils anliegt.

Das in Figur 10 dargestellte Stechgerät 20 bildet sich zusammen mit einem anhand der Figuren 1 bis 9 beschriebenen Magazin 1 ein Stechsystem.

### Bezugszahlen

- 1: Magazin
- 2: Rolle
- 3: Rolle
- 4: Lanzettenträger
- 5: Lanzette
- 6: Testfeld
- 7: Kopplungseinrichtung
- 8: Aufnahme für Lanzettenträger
- 9: Kopplungselement / Borsten
- 10: Schlitten
- 11: Stechantrieb
- 12: Andruckelement
- 13: Drehpunkt
- 14: Federelement
- 15: Kulisse
- 16: Kopplungsfläche
- 17: Öffner
- 20: Stechgerät
- 21: Geräteöffnung
- 22: Bedienungselement
- 23: Anzeigeeinrichtung

## Patentansprüche

1. Magazin für ein Stechgerät zum Gewinnen einer Körperflüssigkeitsprobe, wobei das Magazin (1) einen bandförmigen Lanzettenträger (4), der mehrere Lanzetten (5) trägt, enthält und in ein Stechgerät einsetzbar ist, das einen Stechantrieb (11) zum Beschleunigen der in dem Magazin (1) enthaltenen Lanzetten (5) für eine Einstichbewegung enthält, wobei das Magazin (1) eine Kopplungseinrichtung (7) zum Koppeln des Lanzettenträgers (4) mit einem Stechantrieb (11) eines Stechgeräts aufweist, wobei die Kopplungseinrichtung (7) eine relativ zu einem Magazingehäuse (11) bewegliche Aufnahme (8) für den Lanzettenträger (4) aufweist, die dafür eingerichtet ist, bei einem Stich eine von einem ankoppelbaren Stechantrieb (11) erzeugte Antriebskraft auf eine in einer Stechposition positionierte Lanzette (5) zu übertragen, **dadurch gekennzeichnet, dass** die Lanzetten (5) quer zur Längsrichtung des Lanzettenträgers (4) orientiert sind und die Aufnahme (8) Teil eines in Einstichrichtung relativ zu dem Magazingehäuse (11) verfahrbaren Schlittens ist.

2. Magazin nach Anspruch 1, **dadurch gekennzeichnet, dass** der Lanzettenträger (4) zwischen den Lanzetten (5) angeordnete Testfelder (6) zur Untersuchung einer aus einer Stichwunde gewonnenen Körperflüssigkeitsprobe trägt.

3. Stechsystem zum Gewinnen einer Körperflüssigkeitsprobe umfassend mindestens ein Magazin (1) nach Anspruch 1 oder 2 und ein Stechgerät, das ein Fach für ein derartiges Magazin (1), einen Fortschaltmechanismus, um die in einem in das Fach eingesetzten Magazin (1) enthaltenen Lanzetten (5) nacheinander in eine Stechposition zu bringen, und einen Stechantrieb (11), um eine in der Stechposition positionierte Lanzette (5) für eine Stechbewegung zu beschleunigen, aufweist,
wobei die von dem Lanzettenträger (4) getragenen Lanzetten (5) relativ zu der Aufnahme (8) durch Betätigen des Fortschaltmechanismus beweglich sind, und
wobei die Aufnahme (8) im Betrieb den Stechantrieb (11) mit einer in der Stechposition positionierten Lanzette (5) koppelt, um bei einem Stich eine von dem Stechantrieb (11) erzeugte Antriebskraft auf die in der Stechposition positionierte Lanzette (5) zu übertragen.

4. Stechsystem nach Anspruch 3, **dadurch gekennzeichnet, dass** die Kopplungseinrichtung (7) mit dem Lanzettenträger (4) koppelt und diesen bei einem Stich zusammen mit einer in der Stechposition positionierten Lanzette (5) bewegt.

5. Stechsystem nach Anspruch 4, **dadurch gekennzeichnet, dass** die Kopplungseinrichtung (7) während einer Betätigung des Fortschaltmechanismus mit dem Lanzettenträger (4) gekoppelt bleibt

6. Stechsystem nach einem der Ansprüche 3 bis 5, **dadurch gekennzeichnet, dass** die Kopplungseinrichtung (7) während einer Betätigung des Fortschaltmechanismus mit dem Stechantrieb (11) gekoppelt bleibt.

7. Stechsystem nach einem der Ansprüche 3 bis 6, **dadurch gekennzeichnet, dass** die Kopplungseinrichtung (7) den Lanzettenträger (4) bei einem Stich klemmend fasst und nach einer erfolgten Einstich- und Rückführbewegung wieder frei gibt.

8. Stechsystem nach Anspruch 7, **dadurch gekennzeichnet, dass** die Kopplungseinrichtung (7) ein Federelement (14) enthält, das eine Klemmkraft zum Einklemmen des Lanzettenträgers (4) bewirkt.

9. Stechsystem nach einem der Ansprüche 3 bis 8, **dadurch gekennzeichnet, dass** die Kopplungseinrichtung (7) mit dem Lanzettenträger (4) über eine strukturierte Kopplungsfläche (16) in Kontakt tritt.

10. Stechsystem nach einem der Ansprüche 3 bis 8, **dadurch gekennzeichnet, dass** die Kopplungseinrichtung (7) mit dem Lanzettenträger (4) über eine Kopplungsfläche (16) in Kontakt tritt, die Fasern (9) trägt, die schräg in Richtung einer Rückführbewegung, die eine Lanzette (5) bei einem Stich nach einer Vorschubphase ausführt, geneigt sind.

11. Stechsystem nach Anspruch 9 oder 10, **dadurch gekennzeichnet, dass** die strukturierte Kopplungsfläche (16) einer Relativbewegung zwischen Lanzettenträger (4) und Kopplungsfläche (16) einen von der Richtung der Relativbewegung abhängigen Widerstand entgegensetzt.

## Claims

1. Magazine for a puncturing device for obtaining a sample of body fluid, whereby the magazine (1) contains a ribbon-shaped lancet carrier (4), which carries several lancets (5), and can be inserted into a puncturing device that comprises a puncturing drive (11) for accelerating the lancets (5), which are contained in the magazine (1), for a puncturing motion, wherein the magazine (1) has a coupling facility (7) for coupling the lancet carrier (4) to a puncturing drive (11) of a puncturing device, whereby the coupling facility (7) has a receptacle (8) for the lancet carrier (4), said receptacle (8) being mobile with respect to a magazine housing, wherein the receptacle (8) is configured to transmit a driving force to a lancet (5) positioned in a puncturing position, said driving force being generated by a puncturing drive (11) coupled to the receptacle (8) during a puncture, **characterized in that** the lancets (5) are oriented transverse to the longitudinal direction of the lancet carrier (4) and that the receptacle (8) is part of a sledge that is mobile with respect to the magazine housing in direction of puncturing.

2. Magazine according to claim 1, **characterized in that** the lancet carrier (4) carries test fields (6) for testing a sample of body fluid obtained from a puncturing wound, said test fields being arranged between the lancets (5).

3. Puncturing system for obtaining a sample of body fluid, comprising at least one magazine (1) according to claim 1 or 2 and a puncturing device that has a compartment for such a magazine (1), an incremental advancing mechanism for moving the lancets (5) contained in a magazine (1) inserted in the compartment successively to a puncturing position, and a puncturing drive (11) for accelerating a lancet (5) that is positioned in the puncturing position for a puncturing motion,
whereby the lancets (5) that are carried by the lancet carrier (4) can be moved relative to the receptacle (8) by actuating the incremental advancing mechanism and whereby the receptacle (8), in operation, couples the puncturing drive (11) to a lancet (5) positioned in the puncturing position for transmitting a driving force generated by the puncturing drive (11) during a puncture to the lancet (5) positioned in the puncturing position.

4. Puncturing system according to claim 3, **characterized in that** the coupling facility (7) couples to the lancet carrier (4) and moves it during a puncture in conjunction with a lancet (5) that is positioned in the puncturing position.

5. Puncturing system according to claim 4, **characterized in that** the coupling facility (7) remains coupled to the lancet carrier (4) during an actuation of the incremental advancing mechanism.

6. Puncturing system according to any one of claims 3 to 5, **characterized in that** the coupling facility (7) remains coupled to the puncturing drive (11) during an actuation of the incremental advancing mechanism.

7. Puncturing system according to any one of claims 3 to 6, **characterized in that** the coupling facility (7) grasps the lancet carrier (4) in a clamping manner during a puncture and releases it again after a puncturing and a returning motion are completed.

8. Puncturing system according to claim 7, **characterized in that** the coupling facility (7) contains a spring element (14) that effects a clamping force for clamping the lancet carrier (4).

9. Puncturing system according to any one of claim 3 to 8, **characterized in that** the coupling facility (7) contacts the lancet carrier (4) via a structured contact surface (16).

10. Puncturing system according to any one of claims 3 to 8, **characterized in that** the coupling facility (7) contacts the lancet carrier (4) via a contact surface (16) that carries fibers (9) that are inclined obliquely in the direction of a returning motion that is performed by a lancet (5) after an advancing phase during a puncture.

11. Puncturing system according to claim 9 or 10, **characterized in that** the structured coupling surface (16) poses a resistance against a relative motion of the lancet carrier (4) and the coupling surface (16) with respect to each other, whereby said resistance depends on the direction of the relative motion.

## Revendications

1. Chargeur pour un appareil de piqûre destiné à prélever un échantillon de liquide corporel, le chargeur (1) contenant un porte-lancettes (4) en forme de bande, qui porte plusieurs lancettes (5), et pouvant être inséré dans un appareil de piqûre qui comporte un mécanisme d'entraînement (11) destiné à accélérer les lancettes (5) contenues dans le chargeur (1) pour qu'elles effectuent un mouvement de piqûre, le chargeur (1) comportant un dispositif de couplage (7) destiné à coupler le porte-lancettes (4) à un mécanisme d'entraînement (11) d'un appareil de piqûre, le dispositif de couplage (7) comportant un logement (8) mobile par rapport à un boîtier de chargeur (11) pour le porte-lancettes (4), qui est conçu pour transmettre, lors d'une piqûre, une force motrice produite par un mécanisme d'entraînement (11) pouvant être accouplé, sur une lancette (5) placée en position de piqûre, **caractérisé en ce que** les lancettes (5) sont orientées transversalement au sens longitudinal du porte-lancettes (4) et le logement (8) constitue une partie d'un chariot pouvant être déplacé par rapport au boîtier de chargeur (11) dans le sens de la piqûre.

2. Chargeur selon la revendication 1, **caractérisé en ce que le** porte-lancettes (4) porte des zones de test (6) disposées entre les lancettes (5) pour l'analyse d'un échantillon de liquide corporel prélevé sur une incision.

3. Système de piqûre destiné à prélever un échantillon de liquide corporel, comprenant au moins un chargeur (1) selon la revendication 1 ou 2 et un appareil de piqûre présentant un compartiment pour un tel chargeur (1), un mécanisme d'avance destiné à amener les lancettes (5), contenues dans un chargeur (1) inséré dans le compartiment, les unes après les autres dans une position de piqûre, et un mécanisme d'entraînement (11) destiné à accélérer une lancette (5) placée en position de piqûre pour qu'elle effectue un mouvement de piqûre,
les lancettes (5) portées par le porte-lancettes (4) étant mobiles par rapport au logement (8) par actionnement du mécanisme d'avance, et
le logement (8), en fonctionnement, couple le mécanisme d'entraînement (11) à une lancette (5) placée en position de piqûre afin de transmettre, lors d'une piqûre, une force motrice produite par le mécanisme d'entraînement (11) sur la lancette (5) placée en position de piqûre.

4. Système de piqûre selon la revendication 3, **caractérisé en ce que** le dispositif de couplage (7) se couple au porte-lancettes (4) et, lors d'une piqûre, déplace celui-ci conjointement avec une lancette (5) placée en position de piqûre.

5. Système de piqûre selon la revendication 4, **caractérisé en ce que,** pendant un actionnement du mécanisme d'avance, le dispositif de couplage (7) reste couplé au porte-lancettes (4).

6. Système de piqûre selon l'une des revendications 3 à 5, **caractérisé en ce que,** pendant un actionnement du mécanisme d'avance, le dispositif de couplage (7) reste couplé au mécanisme d'entraînement (11).

7. Système de piqûre selon l'une des revendications 3 à 6, **caractérisé en ce que,** lors d'une piqûre, le dispositif de couplage (7) saisit le porte-lancettes (4) avec serrage et le relâche une fois qu'un mouvement de piqûre et de rappel a eu lieu.

8. Système de piqûre selon la revendication 7, **caractérisé en ce que** le dispositif de couplage (7) comporte un élément faisant ressort (14) qui produit une force de serrage pour serrer le porte-lancettes (4).

9. Système de piqûre selon l'une des revendications 3 à 8, **caractérisé en ce que** le dispositif de couplage (7) vient en contact avec le porte-lancettes (4) par l'intermédiaire d'une surface de couplage (16) structurée.

10. Système de piqûre selon l'une des revendications 3 à 8, **caractérisé en ce que** le dispositif de couplage (7) vient en contact avec le porte-lancettes (4) par l'intermédiaire d'une surface de couplage (16) portant des fibres (9) inclinées dans la direction d'un mouvement de rappel que réalise une lancette (5) lors d'une piqûre après une phase d'avance.

11. Système de piqûre selon la revendication 9 ou 10, **caractérisé en ce que** la surface de couplage (16) oppose à un mouvement relatif entre porte-lancettes (4) et surface de couplage (16) une résistance qui dépend de la direction du mouvement relatif.
